# EUROPÄISCHE PATENTSCHRIFT

(11) **EP 1 362 586 B1**
(45) Veröffentlichungstag und Bekanntmachung des Hinweises auf die Patenterteilung: **07.09.2005**
(21) Anmeldenummer: 03450035.5
(22) Anmeldetag: 05.02.2003
(51) Int. Cl.: A61K 31/194, C12P 13/14, A61K 31/11, A61K 31/34, A61P 25/32, A61P 25/34

(54) **Mittel zur Raucher-Entwöhnung oder zur Alkohol-Entwöhnung**
Composition for the treatment of alcohol and smoking withdrawal
Moyen pour le traitement de sevrage de l'alcool et de cigarettes

(30) Priorität: 17.05.2002 AT 7642002
(43) Veröffentlichungstag der Anmeldung: 19.11.2003
(73) Patentinhaber: Groke, Karl, 8063 Eggersdorf (AT); Kager, Ernst, 8141 Unterpremstätten (AT); Bücherl, Christian, Ing., 8501 Lieboch (AT)
(72) Erfinder: Groke, Karl, 8063 Eggersdorf (AT); Kager, Ernst, 8141 Unterpremstätten (AT); Bücherl, Christian, Ing., 8501 Lieboch (AT)
(74) Vertreter: Müllner, Erwin

(56) Entgegenhaltungen:
- GRIFFATON G ET AL: "EFFECTS OF A SALT OF ALPHA-KETO GLUTARIC-ACID ON THE METABOLIC DISTURBANCES CAUSED BY ETHANOL IN RATS" ARCHIVES DES SCIENCES PHYSIOLOGIQUES, Bd. 26, Nr. 1, 1972, Seiten 69-83, XP008020744 ISSN: 0003-9713
- TREMOLIERES J ET AL: "Role of different amino acids in ethanol intoxication" REVUE DE L'ALCOOLISME 1973, Bd. 19, Nr. 2, 1973, Seiten 107-114, XP001164105
- RAWAT A K: "Neurochemical consequences of ethanol on the nervous system" INTERNATIONAL REVIEW OF NEUROBIOLOGY 1976, Bd. 19, 1976, Seiten 123-172, XP008020344

## Beschreibung

Aus der AT 393221 B ist bekannt, dass alpha-Ketoglutarsäure und 5-Hydroxymethylfurfural eine zerstörende Wirkung auf malignc Tumore haben.

Es wurde nun überraschender Weise gefunden, dass sich dieses Mittel hervorragend dazu eignet, sich das Rauchen oder übermäßigen Alkoholkonsum abzugewöhnen. Besonders hervorzuheben ist, dass dieses Mittel nahezu keine unerwünschten Wirkungen entfaltet, lediglich ein verstärkter Harndrang wurde beobachtet.

Die vorliegende Erfindung sieht daher die Verwendung von alpha-Ketoglutarsäure und 5-Hydroxymethylfurfural zur Herstellung eines Mittels zur Raucher-Entwöhnung oder zur Alkohol-Entwöhnung vor.

Vorzugsweise beträgt das Masseverhältnis von alpha-Ketoglutarsäure zu 5-Hydroxymethylfurfural 2:1 bis 12:1.

Gemäß der AT 393221 B wird das Mittel als Infusion verabreicht. Für die Entwöhnung vom Rauchen oder von übermäßigem Alkoholkonsum ist eine Formulierung als Getränk vorzuziehen. Gegenstand der Erfindung ist somit auch ein Getränk, das folgende Bestandteile pro Liter enthält:
4-8g alpha-Ketoglutarsäure
0,2-0,6g 5-Hydroxymethylfurfural
20-40g Sacharose
2,5-5g Natriumbicabonat
0,3-0,8g Sorbinsäure
gegebenenfalls bis 0,5g Zitronensäure.

Der pH-Wert beträgt vorzugsweise 4-5.

Dieses Getränk hat einen guten Geschmack, wie ein Erfrischungsgetränk.

Günstig erweist sich dabei die stimmungsaufhellende Wirkung von 5-Hydroxymethylfurfural. Das Getränk wird daher gerne regelmäßig konsumiert, was für einen Erfolg von entscheidender Bedeutung ist.

Alpha-Ketoglutarsäure und 5-Hydroxymethylfurfural sind Radikalfänger, was möglicherweise die Ursache für die erfindungsgemäße Wirkung ist. Diese Eigenschaft ist aus der AT 393221 B nicht bekannt. Radikale wurden bisher schon in Zusammenhang mit der Entstehung maligner Tumore gebracht. Beim Rauchen rechnet man mit 10¹⁴ freien Radikalen pro Zug. Auch übermäßiger Alkoholkonsum liefert Radikale. Es ist denkbar, dass durch die radikalfangende Wirkung die Lust auf die Zigarette bzw. Alkohol verringert wird, weil diese Wirkung der Zigarette bzw. des Alkohols (Erzeugung freier Radikale) ausbleibt.

Folgendes Getränk wurde an verschiedene Versuchspersonen, die alle Raucher waren (20 bis 40 Zigaretten täglich), ausgegeben (alle Angaben beziehen sich auf 1 Liter):
5g alpha-Ketoglutarsäure
0,5g 5-Hydroxymethylfurfural
30g Sacharose
3,66g Natriumbicabonat
0,5g Sorbinsäure
0,5g Zitronensäure.
Der pH-Wert war etwa 4,5.

Die Versuchspersonen bekamen dieses Getränk in ausreichender Menge zur Verfügung gestellt. Sie tranken 11 pro Tag, gleichmäßig verteilt auf 12 Stunden, immer auf nüchternen Magen. Nach einer Woche zeigte sich folgendes Ergebnis:
etwa 25%: keine Wirkung auf das Rauchen
etwa 25%: weniger Lust auf Rauchen, aber unveränderter Zigarettenkonsum
etwa 25%: weniger Lust auf Rauchen, Zigarettenkonsum auf weniger als die Hälfte verringert (aber immer noch vorhanden)
etwa 25%: kein Ziqarettenkonsum mehr.

Es wurde auch folgendes Getränk getestet:
6g alpha-Ketoglutarsäure
0,4g 5-Hydroxymethylfurfural
40g Sacharose
4,2g Natriumbicabonat
0,6g Sorbinsäure
0,2g Zitronensäure.
Der pH-Wert war etwa 4,5.

Die Wirkung war ähnlich, die beiden Getränke haben lediglich einen etwas unterschiedlichen Geschmack.

## Patentansprüche

1. Verwendung von alpha-Ketoglutarsäure und 5-Hydroxymethylfurfural zur Herstellung eines Mittels zur Raucher-Entwöhnung oder zur Alkohol-Entwöhnung.

2. Verwendung nach Anspruch 1, **dadurch gekennzeichnet, dass** das Masseverhältnis von alpha-Ketoglutarsäure zu 5-Hydroxymethylfurfural 2:1 bis 12:1 beträgt.

3. Getränk, **dadurch gekennzeichnet, dass** es folgende Bestandteile pro Liter enthält:
4-8g alpha-Ketoglutarsäure
0,2-0,6g 5-Hydroxymethylfurfural
20-40g Sacharose
2,5-5g Natriumbicabonat
0,3-0,8g Sorbinsäure
gegebenenfalls bis 0,5g Zitronensäure.

4. Getränk nach Anspruch 4, **dadurch gekennzeichnet, dass** der pH-Wert 4-5 beträgt.

## Claims

1. Use of alpha-ketoglutaric acid and 5-hydroxymethylfurfural in the manufacture of an agent for withdrawal from smoking or alcohol.

2. Use according to claim 1, **characterised in that** the mass ratio of alpha-ketoglutaric acid and 5-hydroxymethylfurfural is from 2:1 to 12:1.

3. A beverage, **characterised in that** it contains the following components per litre:
4 - 8g alpha-ketoglutaric acid
0.2 - 0.6g 5-hydroxymethylfurfural
20 - 40g sucrose
2.5 - 5g sodium carbonate
0.3 - 0.8g sorbic acid
where required up to 0.5g citric acid

4. A beverage according to claim 4, **characterised in that** the pH-value is 4 - 5.

## Revendications

1. Application d'acide α-cétoglutamique et de 5-hydroxyméthylfurfural pour la fabrication d'un moyen de sevrage de l'alcool et du tabac.

2. Application selon la revendication 1, **caractérisée en ce que** le rapport massique α-cétoglutamique/5-hydroxyméthylfurfural est compris entre 2/1 et 12/1.

3. Boisson, **caractérisée en ce qu'**elle contient par litre :
- 4,8 g d'α-cétoglutamique ;
- 0,2 à 0,6 g de 5-hydroxyméthylfurfural ;
- 20 à 40 g de saccharose ;
- 2,5 à 5 g de carbonate de sodium ;
- 0,3 à 0,8 g d'acide sorbique
et le cas échéant jusqu'à 0,5g d'acide citrique

4. Boisson selon la revendication 3, **caractérisée en ce que** son pH est compris entre 4 et 5.
